# EUROPEAN PATENT SPECIFICATION

(11) **EP 1 393 755 B1**
(45) Date of publication and mention of the grant of the patent: **14.12.2005**
(21) Application number: 03077564.7
(22) Date of filing: 15.08.2003
(51) Int. Cl.: A61L 9/03, H01R 13/447

(54) **Discrete vapor-dispensing device and refill**
Unauffälliger Verdampfer und Nachfüllpackung
Vaporisateur discret et cartouche

(30) Priority: 16.08.2002 US 222079; 12.02.2003 WO PCT/US03/04082
(43) Date of publication of application: 03.03.2004
(73) Proprietor: The Dial Corporation, Scottsdale, AZ 85260-1619 (US)
(72) Inventor: He, Mengtao Pete, Scottsdale, Arizona 85259 (US); Triplett, Carl, Scottsdale, Arizona 85260 (US); Conway, Mary J., Phoenix, Arizona 85022 (US)
(74) Representative: Moore, Barry

(56) References cited:
- EP-A- 0 296 807
- WO-A-01/10739
- WO-A-01/68154
- US-A- 5 574 821

## Description

### BACKGROUND OF THE INVENTION

This invention relates, generally, to vapor-dispensing devices and, in particular, to a vapor-dispensing device which integrates with a receptacle in a discrete manner.

### TECHNICAL FIELD

It is often convenient to use an available receptacle, for example, a conventional household electrical outlet, as a source of electrical power for systems configured to perform some type of environment-altering task. Known environment-altering systems include, for example, plug-in air-fresheners, vapor-dispensing devices, plug-in ultrasonic pest control devices, night-lights, ionizers, and the like.

Due to the location and accessibility of conventional wall outlets, and the size and shape of known vapor-dispensing devices, it is common for such devices to be relatively conspicuous when plugged into a wall outlet. In the case of household plug-in air-fresheners, for example, this may give rise to unfortunate conclusions in the minds of visitors with respect to the overall quality of air in the home.

In some instances it may therefore be desirable to enhance the extent to which vapor-dispensing devices integrate or blend in with their environment. The level of discreetness is generally related to a device's overall geometry and the manner in which the device contacts the wall and/or receptacle to which it is connected.

In addition to this *aesthetic* discreetness, it would also be advantageous to provided an environment-altering apparatus with enhanced *functional* discreetness. That is, there is a need for vapor-dispensing devices which do not eliminate, reduce, or otherwise alter a user's access to the functional features of the receptacle. In the case of plug-in air-fresheners, for example, it would be advantageous to provide an air-freshener wherein access to all or most of the receptacle's outlets are substantially maintained.

Examples of known aroma diffuser assemblies include EP 0296807 and WO 01/68154. The former provides a housing that includes a portion for connection to an external power source. It also includes a second portion for supplying power through the housing to an external power consuming device. The latter provides an air freshener having a plug-in capability as well as a night-light.

### SUMMARY OF THE INVENTION

In general, the present invention provides environment-altering apparatus according to claim 1. Advantageous embodiments are provided in the dependent claims.

In accordance with one embodiment of the present invention, an environment-altering apparatus is configured to mimic an electrical receptacle having an outlet pattern. The environment-altering apparatus has a front surface which includes an outlet pattern substantially corresponding to the outlet pattern of the electrical receptacle, and a plug configured to interface with the electrical receptacle. A device configured to modify one or more attributes of the environment is interposed between the front surface and plug pattern. These attributes include, for example, aromatic vapor density, insecticide vapor density, ambient light intensity, ionic air content, ultrasonic frequency intensity, and the like.

### BRIEF DESCRIPTION OF THE DRAWINGS

The subject invention will hereinafter be described in conjunction with the appended drawing figures, wherein like numerals denote like elements, and:
FIG. 1 is a schematic overview of a system providing a context in which the present invention may be practiced;
FIG. 2 shows a schematic side view of a vapor-dispensing device and receptacle in accordance with the present invention;
FIG. 3 is a schematic front view of an exemplary receptacle having a number of outlets;
FIG. 4 depicts a conventional dual-outlet receptacle;
FIG. 5 depicts a conventional quad-outlet receptacle;
FIG. 6 illustrates a variety of exemplary receptacle and outlet configurations;
FIG. 7 is an isometric view showing exemplary vapor-dispensing device geomeches; and
FIGS. 8A-8C depict various refill geometries in accordance with one aspect of the present invention.

### DETAILED DESCRIPTION

Systems in accordance with the present invention generally provide a vapor-dispensing device or other environment-altering apparatus configured to integrate with its environment in a discreet manner.

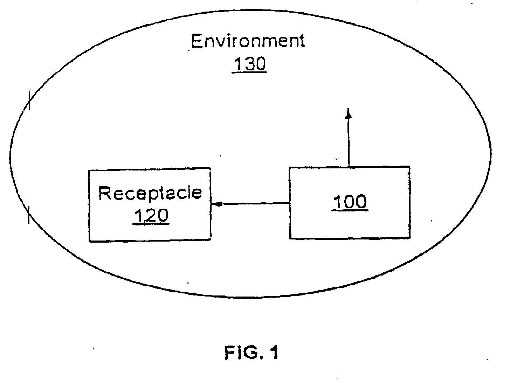

Referring to Fig. 1, an environmental-altering apparatus 100 in accordance with the present invention generally interfaces with a receptacle 120 within an environment 130. Environmental-altering apparatus 100 comprises any suitable component or combination of components configured to alter environment 130 in some manner, e.g., by altering its aromatic vapor density, dispensing an insecticide, providing a light source, ionizing the ambient air, and/or providing a source of ultrasonic sound. In this regard, the phrase "vapor-dispensing device" may, without loss of generality, be used herein to refer to the environment-altering apparatus. Those skilled in the art will recognize that the present invention is not so limited.

Environment 130 corresponds to any defined space, whether open or enclosed by one or more surfaces, walls, ceilings, floors, or other solid or fictitious boundaries, which receives the evaporated material. For example, environment 130 may correspond to a residential room (bedroom, bathroom, kitchen, etc.), commercial space (factory floor, office cubicles, etc.), automotive enclosure (car, truck, recreational-vehicle), airline compartment, or any other space in which it is desirable to deliver a vapor.

In accordance with one embodiment of the present invention, an environment-altering apparatus is configured to give the appearance that it is something other than an environment-altering apparatus. For example, the environment-altering apparatus may be configured to mimic an electrical receptacle having an outlet pattern. In this embodiment, the environment-altering apparatus has a front surface which includes an outlet pattern substantially corresponding to the outlet pattern of the receptacle, and a plug configured to interface with the electrical receptacle.

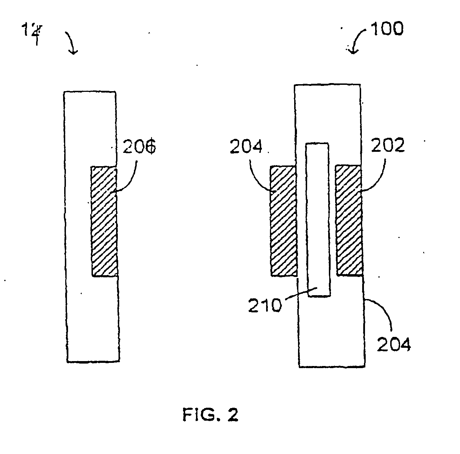

For example, referring now to Fig. 2, a vapor dispensing device 100 interfaces with a receptacle 120 through one or more plugs (or other interface structures) corresponding to an outlet pattern 206. Vapor dispensing device 100 has a front surface 204 (which may or may not be planar) that includes an outlet pattern 202 which substantially corresponds to outlet pattern 206 of receptacle 120. Thus, vapor dispensing device 100 is generally configured to removeably attach to receptacle 120.

A device 210 configured to modify one or more attributes of the environment is suitably interposed between (and/or to the side of) the front surface 204 and plug pattern 204. Environmental attributes modified by device 210 include, for example, aromatic vapor density, insecticide vapor density, ambient light intensity, ionic air content, ultrasonic frequency intensity, and the like. In an embodiment wherein device 210 corresponds to a fragrance delivery device, it may also include one or more components (e.g., wicks, capillary tubes, and the like) which provide a means for at transporting volatizable material from one location to another (e.g., from a reservoir to a evaporation pad or eminator), and/or one or more components (eminator pads, secondary wicks, and the like) which provide a surface or surfaces from which the transported material undergoes mass transfer or evaporation to environment 120.

In an embodiment wherein receptacle 120 corresponds to an electrical power outlet, it is advantageous to utilize this power source to provide any electrical functionality required by the fragrance delivery device. For example, fragrance delivery device may include one or more eminators or heating elements designed to control the rate at which the volatizable material evaporates into the environment. In such a case, delivery device 210 may include various terminals, wires, conductive traces, plugs, and other such components facilitating interface and power delivery to receptacle 120. In a particularly preferred embodiment, for example, delivery device 210 includes a resistive heating element that is thermally coupled to an eminator pad or wick which communicates with a volatizable material.

Although the vapor dispensing device 100 of Fig. 2 is illustrated as generally rectilinear in cross-section, it may in fact include any number of discrete or integrated housings having any arbitrary shape.

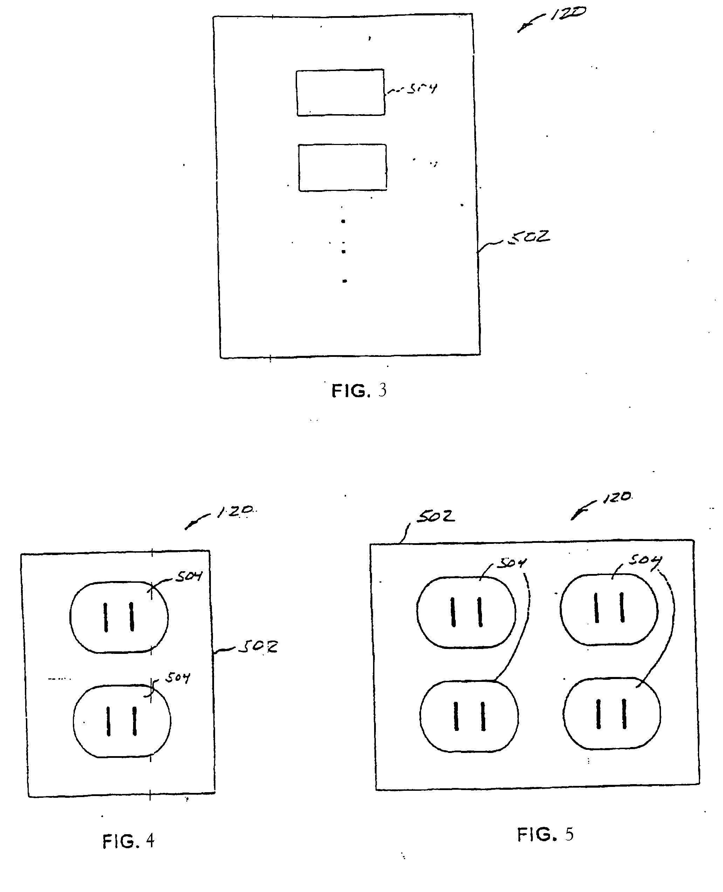

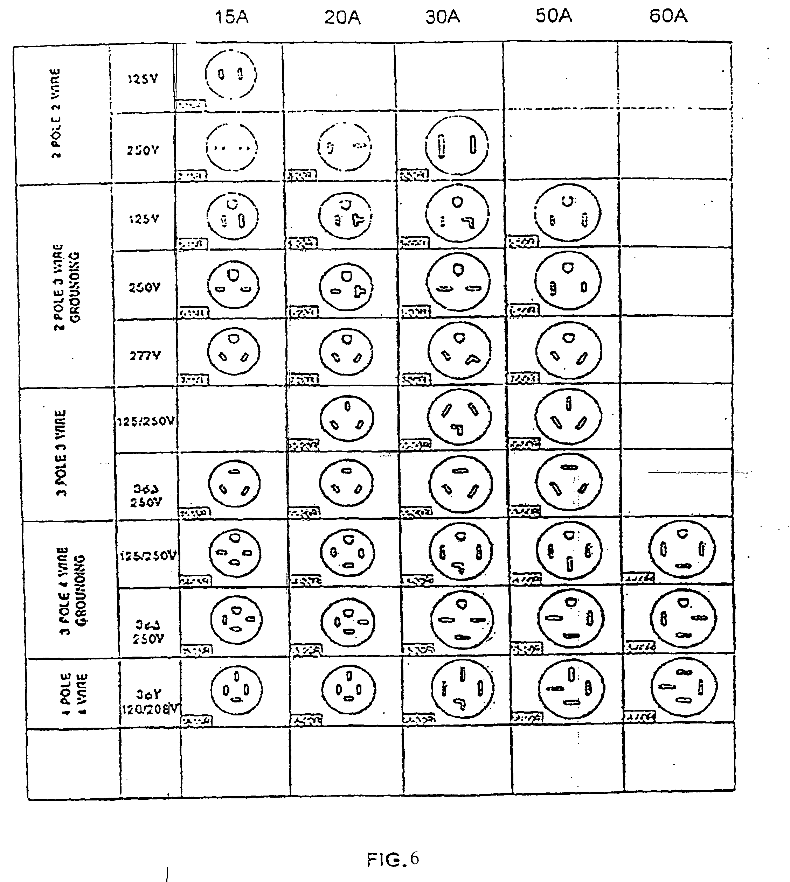

Fig. 3 shows a general configuration for receptacle 120 which includes an outer boundary and/or faceplate 502 along with one or more outlets 504. Outlets 504 may exhibit any suitable shape, and may include any suitable combination of male, female, or other connection types. For example, referring now to Fig. 4, receptacle 120 may consist of a conventional dual-outlet power receptacle including a pair of two-prong outlets 504 and a faceplate 502. Similarly, as shown in Fig. 5, receptacle 120 may consist of a conventional quad-outlet power receptacle including four two-prong outlets 504 and a faceplate 502. Fig. 6 presents a matrix of additional standard receptacle designs with which the present invention may be employed. Note also that the present invention may be used in connection with ground-fault interrupt (GFI) electrical outlets.

Notwithstanding the nature of receptacle 120 -- i.e., whether and to what extent receptacle 120 is configured to supply electrical current -- delivery device 210 may be passive, active, or selectably switched between active and passive modes. The term "passive" in this context, as applied to delivery devices, refers to those devices which substantially depend upon ambient conditions to deliver a fragrance or otherwise give rise to a modification of the environment. Such ambient conditions include, for example, ambient thermal conditions (e.g., wall surface temperature and air temperature) and ambient air flow, (e.g., air flow resulting from free convection as well as the movement (if any) of fans, individuals, and other entities within the environment).The term "active" in this context refers to devices that are not passive, e.g., devices which employ integrated fans, heating elements, and other such devices.

In the event that delivery device 210 is an active device, any power source required by the device may be intrinsic to receptacle 120, e.g., the 120 V source of a standard wall outlet, or extrinsic to receptacle 120, e.g., supplied by a battery, solar cell, or other such device incorporated into or otherwise associated with delivery device 210. Alternatively, power may be supplied by a combination of intrinsic and extrinsic sources and/or may be incorporated into a refill component, described in further detail below.

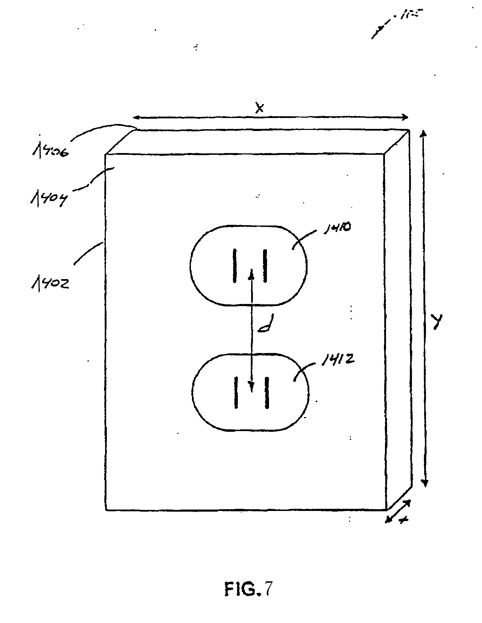

In accordance with a further aspect of the present invention, a vapor-dispensing device has low-profile dimensions. More particularly, referring now to Fig. 7, an exemplary vapor-dispensing device 100 includes a housing 1402 having a front surface 1404 and a back surface 1406 separated by a thickness t, wherein back surface 1406 has a minor axis dimension x and a major axis dimension y. A first device outlet 1410 and a second device outlet 1412 are provided on the front surface 1404 of housing 1402. The device outlets (1410 and 1412) are separated by an inter-outlet distance d; wherein: x/d is between approximately 2.0 and 2.5, preferably about 2.125, y/d is between approximately 3.0 and 3.5, preferably about 3.25, and t/d is between approximately 0.5 and 1.0, preferably about 0.625 In accordance with yet a further aspect of the present invention, the thickness t is less than approximately 20% of the major axis dimension y.

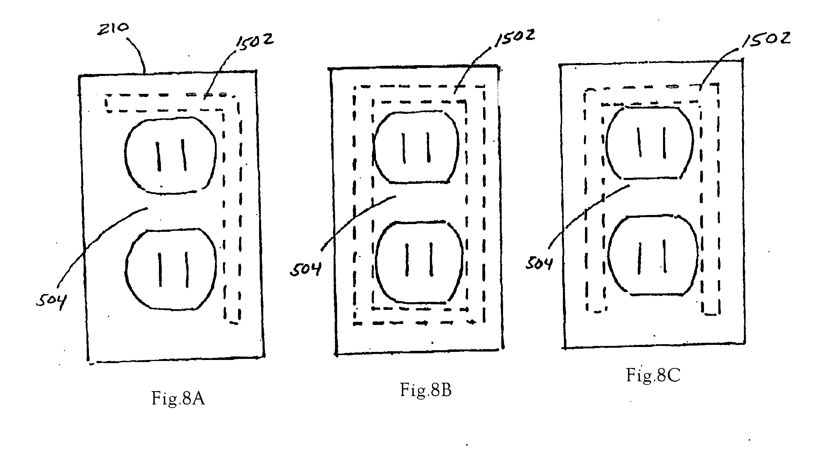

Delivery device 210 suitably includes one or more removeably attached refill components. That is, referring to Figs. 8A-8C, it may be advantageous for delivery device 210 to include components that are integral to the delivery device itself as well as one or more refill components 1502 (or simply "refills") that can be replaced by the user. In the event delivery device 210 is an air freshener device, for example, a depleted refill component 1502 may removed from device 210 and replaced by a new refill containing fragrant oil, wax, gel, or the like. The refill suitably includes a refill body and a volatizable material provided therein.

In accordance with one aspect of the present invention, a refill component is provided which allows vapor-dispensing device to mimic an electrical receptacle. For example, a refill component comprising a refill body having a volatizable material provided therein may be configured to be inserted behind the front surface of the device such that it is substantially concealed by the front surface. In accordance with one aspect of the present invention, the refill has a perimeter that is encompassed by the perimeter of the housing.

In accordance with another aspect of the present invention, the refill is configured such that it does not significantly obstruct the receptacle's outlet pattern 504. In one embodiment, for example, this is accomplished by providing a refill component 1502 that at least partially surrounds one or more outlets on the receptacle (variously shown in Figs. 8A-8C). In the event that the delivery device is used in connection with a standard electrical receptacle, it is desirable for refill 1502 to encompass two or more sides of the outlet pattern (Fig. 8A). To the extent that it is advantageous to supply the greatest possible volume of volatizable material, the refill may be configured as a rectangular ring that completely surrounds the outlet pattern 504 (Fig. 8B). Alternatively, the refill may be configured in a 'U' $hape to allow refill 1502 to be slideably removed from the device (Fig. 8C).

## Claims

1. An environment-altering apparatus 100 for connection to an electrical wall outlet 120, said electrical wall outlet having a first outlet 206, the apparatus comprising:
a front surface 204 having a first device outlet 202 substantially corresponding to the first outlet 206;
a back surface and an electrical plug extending from said back surface for connecting to the first outlet ; and
a device 210 interposed between said front surface and said plug , said device configured to modify an attribute of the environment, wherein said attribute is selected from the group consisting of aromatic vapor density, insecticide vapor density, ambient light intensity, ionic air content, and ultrasonic frequency intensity, and **characterised in that** the apparatus is configured to mimic a conventional electrical wall outlet 120.

2. The apparatus of claim 1, wherein said device 210 is a passive device.

3. The apparatus of claim 1, wherein said device 210 is an active device.

4. The apparatus of claim 1, further comprising a refill component 1502 provided between said front surface and said plug.

5. The apparatus of any preceding claim, wherein said front surface has a second device outlet, the plug being electrically coupled to at least one of the first and second device outlets.

6. The apparatus of claim 5 wherein said first and second device outlets are separated by an inter-outlet distance *d, the front surface and back* surface being separated by a thickness *t*, wherein said back surface has a minor axis dimension *x* and a major axis dimension *y*;
wherein:
*x*/*d* is between approximately 2.0 and 2.5;
*y*/*d* is between approximately 3.0 and 3.5;
*t*/*d* is between approximately 0.5 and 1.0.

7. The apparatus of claim 6, said apparatus being a vapor-dispensing device and further comprising a refill component 1502, said refill component comprising:
a refill body having a thickness less than t, said refill body removeably connected to said vapor-dispensing device.

8. The apparatus of claim 7, wherein said refill component at least partially surrounds said first and second outlets.

9. The apparatus of claim 7, wherein said refill is generally "U"-shaped.

10. The apparatus of claim 7, wherein the refill is configured to slideably attach to the environment-altering apparatus.

11. The apparatus of claim 7, wherein said refill is a rectangular ring.

## Patentansprüche

1. Umgebungsverändernde Vorrichtung 100 zum Anschließen an eine elektrische Steckdose 120, wobei die elektrische Steckdose einen ersten Stromabgabepunkt 206 aufweist, wobei die Vorrichtung Folgendes umfasst:
eine Vorderseite 204 mit einer ersten Vorrichtungs-Auslassstelle 202, die im Wesentlichen dem ersten Stromabgabepunkt 206 entspricht;
eine Rückseite und einen elektrischen Stecker, der sich von der Rückseite erstreckt, zum Anschließen an den ersten Stromabgabepunkt 206; und
eine Vorrichtung 210, die zwischen der Vorderseite und dem Stecker angeordnet ist, wobei die Vorrichtung so konfiguriert ist, dass sie ein Attribut der Umgebung verändert, wobei das Attribut ausgewählt ist aus der Gruppe bestehend aus: Duftdampfdichte, Insektiziddampfdichte, Umgebungslichtintensität, Ionengehalt in der Luft sowie Ultraschallfrequenzintensität, und **dadurch gekennzeichnet, dass** die Vorrichtung so konfiguriert ist, dass sie eine herkömmliche Steckdose 120 nachahmt.

2. Vorrichtung nach Anspruch 1, wobei die Vorrichtung 210 eine passive Vorrichtung ist.

3. Vorrichtung nach Anspruch 1, wobei die Vorrichtung 210 eine aktive Vorrichtung ist.

4. Vorrichtung nach Anspruch 1, die des Weiteren eine Nachfüllkomponente 1502 umfasst, die zwischen der Vorderseite und dem Stecker angeordnet ist.

5. Vorrichtung nach einem der vorangehenden Ansprüche, wobei die Vorderseite eine zweite Vorrichtungs-Auslassstelle aufweist, wobei der Stecker mit der ersten Vorrichtungs-Auslassstelle oder der zweiten Vorrichtungs-Auslassstelle oder mit beiden Vorrichtungs-Auslassstellen elektrisch verbunden ist.

6. Vorrichtung nach Anspruch 5, wobei die erste und die zweite Vorrichtungs-Auslassstelle um einen Abstand d zwischen den Auslassstellen voneinander getrennt sind, wobei die Vorderseite und die Rückseite um eine Dicke t voneinander getrennt sind, wobei die Rückseite eine Nebenachsenabmessung x und eine Hauptachsenabmessung y aufweist;
wobei:
x/d zwischen etwa 2,0 und 2,5 liegt;
y/d zwischen etwa 3,0 und 3,5 liegt;
t/d zwischen etwa 0,5 und 1,0 liegt;

7. Vorrichtung nach Anspruch 6, wobei die Vorrichtung ein Verdampfer ist und des Weiteren eine Nachfüllkomponente 1502 umfasst, wobei die Nachfüllkomponente Folgendes umfasst:
einen Nachfüllkörper mit einer Dicke von weniger als t, wobei der Nachfüllkörper lösbar mit dem Verdampfer verbunden ist.

8. Vorrichtung nach Anspruch 7, wobei die Nachfüllkomponente die erste und die zweite Auslassstelle wenigstens teilweise umgibt.

9. Vorrichtung nach Anspruch 7, wobei die Nachfüllpackung allgemein "U"-förmig ist.

10. Vorrichtung nach Anspruch 7, wobei die Nachfüllpackung so konfiguriert ist, dass sie auf die umgebungsverändernde Vorrichtung aufgeschoben werden kann.

11. Vorrichtung nach Anspruch 7, wobei die Nachfüllpackung ein rechtwinkliger Ring ist.

## Revendications

1. Un appareil modificateur de l'environnement 100 à connecter à une sortie murale électrique 120, ladite sortie murale électrique comprenant une première sortie 20, l'appareil comportant :
une surface frontale 204 ayant une première sortie d'appareil 202 correspondant sensiblement à la première sortie 206,
une surface arrière et une prise électrique s'étendant depuis ladite surface arrière à connecter à la première sortie et
un dispositif 210 interposé entre ladite surface frontale et ladite prise, ledit dispositif étant conçu pour modifier un attribut de l'environnement, ledit attribut étant sélectionné dans le groupe composé de la densité de vapeur, de la densité de vapeur insecticide, de l'intensité lumineuse ambiante, du contenu ionique de l'air et de l'intensité de fréquence ultrasonique, et **caractérisé en ce que** l'appareil est conçu pour imiter une sortie murale électrique conventionnelle 120.

2. Appareil selon la revendication 1, dans lequel ledit dispositif 210 est un dispositif passif.

3. Appareil selon la revendication 1, dans lequel ledit dispositif 210 est un dispositif actif.

4. Appareil selon la revendication 1, comprenant en outre un composant de recharge 1502 installé entre ladite surface frontale et ladite prise.

5. Appareil selon la revendication précédente, dans lequel ladite surface frontale a une seconde sortie d'appareil, la prise étant couplée électriquement à au moins une des première et seconde sorties d'appareil.

6. Appareil selon la revendication 5, dans lequel lesdites première et seconde sorties d'appareil sont séparées par une distance entre sorties d, la surface frontale et la surface arrière étant séparées par une épaisseur t, ladite surface arrière ayant une dimension mineure d'axe x et une dimension majeure d'axe *y*,
sachant que :
*x*/*d* se situe approximativement entre 2,0 et 2,5,
*y*/*d* se situe approximativement entre 3,0 et 3,5,
*t*/*d* se situe approximativement entre 0,5 et 1,0.

7. Appareil selon la revendication 6, ledit appareil étant un appareil dispensateur de vapeur comprenant un composant de recharge 1502, ledit composant de recharge incluant :
un corps de recharge d'une épaisseur inférieure à t, ledit corps de recharge étant connecté de manière amovible audit appareil dispensateur de vapeur.

8. Appareil selon la revendication 7, dans lequel ledit composant de recharge entoure au moins partiellement lesdites première et seconde sorties.

9. Appareil selon la revendication 7, dans lequel ladite recharge est généralement en forme de « U ».

10. Appareil selon la revendication 7, dans lequel la recharge est conçue de manière à se rattacher de manière coulissante à l'appareil modificateur de l'environnement.

11. Appareil selon la revendication 7, dans lequel ladite recharge est une bague rectangulaire.
